# EUROPEAN PATENT SPECIFICATION

(11) **EP 0 496 345 B1**
(45) Date of publication and mention of the grant of the patent: **28.08.1996**
(21) Application number: 92100923.9
(22) Date of filing: 21.01.1992
(51) Int. Cl.: G01N 33/569, C12Q 1/04, C12Q 1/14, C12Q 1/06

(54) **Method for detecting and quantifying cariogenic bacteria**
Verfahren zum Nachweis und zur Quantifizierung kariogener Bakterien
Méthode de détection et quantification de bactéries cariogénique

(30) Priority: 22.01.1991 JP 22858/91; 07.09.1991 JP 227539/91
(43) Date of publication of application: 29.07.1992
(73) Proprietor: NAGASE & COMPANY, LTD., Osaka (JP)
(72) Inventor: Miyazaki, Toshitsugu, Kobe-shi, Hyogo 651-22 (JP); Matsuda, Yoko, Kobe-shi, Hyogo 650 (JP); Nakamura, Tsutomu, Kyoto-shi, Kyoto 606 (JP); Ota, Fusao, Tokushima-shi, Tokushima 770 (JP); Nishino, Mizuho, Tokushima-shi, Tokushima 770 (JP)
(74) Representative: VOSSIUS & PARTNER

(56) References cited:
- WO-A-88/00345
- WO-A-88/06455
- US-A- 4 399 229
- PATENT ABSTRACTS OF JAPAN, vol. 14, no. 446 (C-763)(4389), 25 September 1990/
- PATENT ABSTRACTS OF JAPAN, vol. 14, no. 446 (C-763)(4389), 25 September 1990/

## Description

The present invention relates to a novel method and a kit for detecting and quantifying S. mutans which is the major microorganism for dental caries.

Of various bacteria present in the oral cavity, S. mutans is noted as an important microorganism in dental caries. It is reported that S. mutans is separated only from 23% of plaques in healthy teeth, while it is always separated from carious regions. It is also reported that the rate of existence of S. mutans is proportional to the rate of teeth decayed, missing, and filled (DMFS). Furthermore, it is known that an increased level of S. mutans may precede the development of caries lesions, which has been experimentally confirmed by checking it through the course up to the development of caries at a healthy tooth surface. In addition, it is also reported that caries will possibly be induced when salivary levels of S. mutans exceeded 4.5 x 10⁴ colony-forming units (CFU) per ml.

Recently, it has been reported that Streptococcus mutans is classified into eight of serotypes a to h based on the differences in serological characteristics (Hamada, S. & Slade, H.D., Microbiol. Rev. 44: 331, 1980). Among them, S. mutans having the serotype of c, e or f may be referred to as S. mutans in a narrow sense, that having the serotype of b may be referred to as Streptococcus rattus, that having the serotype of d or g may be referred to as Streptococcus sobrinus, that having the serotype of h may be referred to as Streptococcus downei, and that having the serotype of a may be referred to as Streptococcus cricetus (designation of Streptococcus mutans in the present specification is used in a wide sense, unless otherwise specified). Among them, S. mutans which is most frequently separated from human is that having the serotype of c, and the percentage of its separation reaches 80% of value. S. mutans having the serotype of d, e, f, g, or h are also separated from human (Takamori, K., "Oral Microbiology for Clinician", pp.185-188, Shorin K.K.).

In such circumstances, several methods for examining a dental caries activity have been developed in order to detect a level of cariogenic bacteria in oral cavity, for example, a method for indirectly determining the level of cariogenic bacteria, which comprises collecting dental plaque from a subject, adding it to an ampoule containing a selective medium for the cariogenic bacteria, cultivating it in an incubator maintained at a constant temperature, and quantifying the amount of an organic acid produced by the cariogenic bacteria by means of coloring of a pH indicator contained in the medium.

Another method for quantifying the cariogenic bacteria, which method aims at reductive action possessed by the cariogenic bacteria, comprises cultivating the bacteria at 37°C in a selective medium and measuring the change of the color of the oxidation-reduction indicator resazurin.

Furthermore, a group of Tokyo Dental University has reported a simple method for counting the number of S. mutans in saliva utilizing a selective medium (Matsukubo, T., Shikagakuhou, Vol. 84, No.1, pp.115-117, 1984).

In addition, a simple sampling method such as micro-pipetting (J. Clin. Microbiol. 7: 82-83) or spatula (J. Clin. Microbiol. 9: 584-588) method has been developed for the purpose of measuring the number of S. mutans in saliva based on the number of colonies formed after inoculating saliva directly on mitis-salivarius bacitracin plate.

However, all of these methods require incubation at a constant temperature (usually 37°C) in their detection steps and consume much time in obtaining results, some of which may require several days. In addition, when clinical samples cannot be cultivated immediately, these methods require a medium for transporting the sample, such as VMG II transport medium, because it is necessary to maintain a high survival rate of cariogenic bacteria in the sample until providing it for the cultivation. Furthermore, a selective medium used in these detection methods does not have a complete selectivity to S. mutans which is an important microorganism in dental caries. For example, it has been reported that selectivity of mitis-salivarius medium often used in these methods (Gold, O.G. et al., Arch. Oral Biol. 18: 1357-1364, 1974) and its modified medium is doubtful.

For example, it has been reported that trypan blue contained in mitis-salivarius medium prevents growth of S. mutans (J. Clin. Microbiol. 5: 604-609) and that Chapman terullite solution similarly contained in the medium decreases the number of colonies to be developed (J. Clin. Microbiol. 5: 578-583). It is also reported that an additive such as sulfonamide, bacitracin, polymyxin or sucrose supplemented in some of modified media for the purpose of selectively cultivating S. mutans in such media inhibits growth of S. mutans (J. Clin. Microbiol. 4: 95-98; J. Clin. Microbiol. 5: 578-583). These reports show that selective media of S. mutans currently used are not satisfactory for selectively isolating all of S. mutans (Microbiological Reviews 44: 331-334).

A detection method of S. mutans which utilizes an immunoassay has also been known. Japanese Patent Publication No.250,067/1989 discloses a method for detecting and quantifying S. mutans utilizing reversed passive agglutination reaction, which method involves immobilizing a polyclonal antibody against S. mutans to insoluble latex beads having a particle size of 0.1-100µm and estimating the degree of antigen-antibody reaction on the basis of the degree of agglutination.

However, this method has an inferior reactivity and thus low sensitivity because it relies on the antigen-antibody reaction between two insoluble molecules, a specific antibody immobilized on insoluble particles and suspended bacteria. In order to improve this defect, it is proposed to enhance the sensitivity by treating the bacteria with a proteinase and nitrous acid to solubilize an antigen on the surface of the bacteria. However, the sensitivity obtained by such method is not thoroughly satisfactory.

Furthermore, there arises a problem in specificity when a polyclonal antibody is used. The polyclonal antibody used in this method is generally prepared from a serum derived from a small animal such as rabbit which is immunized with S. mutans itself as an antigen. Accordingly, the polyclonal antibody just contains antibodies against the antigen commonly possessed by S. mutans and another bacteria, and therefore, is inferior in specificity.

The problem of specificity can be solved if a monoclonal antibody is employed. However, its sensitivity is still low so long as a reaction of an immobilized antibody and bacteria is utilized because the reaction is carried out between two insoluble molecules as described above. In addition, the monoclonal antibody reacts with a single type of antigen present on the surface of bacteria in contrast to a polyclonal antibody. Accordingly, it is more difficult to form a cross-linkage between carriers in comparison with the method using a polyclonal antibody, and therefore, an agglutination reaction does not occur or hardly occurs. Thus, in the agglutination method, use of a polyclonal antibody is more advantageous than that of a monoclonal antibody, in a certain aspect.

Among the immunoassays utilizing a monoclonal antibody, an enzyme immunoassay has widely been utilized in clinical diagnosis and used in detection and quantification of biological components. Among the assays, a sandwich method is superior in sensitivity, specificity and quantification, and often employed. This method involves binding of a monoclonal antibody specific to an antigen to an insoluble solid phase, specifically binding the antigen to the antibody, binding thereto further a labelled secondary antibody specific to the antigen, and separating the bound antibody and the free antibody (B/F separation) by means of washing of the solid phase with an appropriate washing solution. However, if the method is applied to the detection of S. mutans, the efficiency of reaction is low because the reaction is an antigen-antibody reaction between two insoluble molecules. Accordingly, B/F separation can not be carried out well, and as a result the sensitivity is very inferior.

WO-A 88/06455 discloses polyclonal and monoclonal antibodies against an antigenic determinant common to *Streptococcus sobrinus* serotype d and *Streptococcus mutans* serotype c and their use to combat dental caries as well as their use in fluid phase radioimmunoassay against whole cells of *Streptococcus mutans.*

WO-A 88/00345 discloses a method and an apparatus for the qualitative and/or quantitative analysis of, e.g. microorganisms whereby a filter is used to separate components contained in the reaction mixture.

As described above, previously known methods for detecting cariogenic bacteria are insufficient and unsuitable in respect of sensitivity, specificity, and quantitative characteristics.

Under the circumstances as described above, the present inventors have extensively investigated for the purpose of developing an improved method for detecting and quantifying cariogenic bacteria, which does not require cultivation of S. mutans in a selective medium and can conveniently detect and quantify S. mutans present in a sample to be examined in a short time with high sensitivity.

As a result, they have found that the desired detection and quantification of cariogenic bacteria can be effected rapidly and conveniently by utilizing a polyclonal or monoclonal antibody specific to the major cariogenic bacteria, S. mutans, and carrying out the B/F separation by means of a membrane.

Thus, the present invention provides a method for detecting and/or quantifying Streptococcus mutans present in a solution to be examined, which method comprises:
(a) dispersing a sample containing S. mutans to be examined in an optimal buffer solution suitable for the antigen-antibody reaction, and adding to the thus prepared suspension a polyclonal or monoclonal antibody having a specific binding ability to S. mutans to allow to carry out the antigen-antibody reaction;
(b) separating an antibody specifically bound to S. mutans (bound antibody) from an antibody not bound to S. mutans (free antibody) in the suspension by filtration through a membrane filter having a pore size capable of capturing S. mutans; and
(c) quantifying the amount of the antibody specifically bound to S. mutans captured on the membrane filter, by a conventional method for quantifying the antibody.

According to the present invention, it is possible to carry out very efficient antigen-antibody reactions between an antigen present on the surface of S. mutans cells and a specific antibody, because a polyclonal or monoclonal antibody is not immobilized on a solid phase and used in a free state or solubilized state. In addition, according to the present invention, because of separating the unreacted specific antibody (B/F separation) by means of filtration through a suitable membrane filter capable of capturing bacterial cells after such efficient antigen-antibody reaction, a higher quantitative immunoassay such as enzyme immunoassay can be applied to the detection of S. mutans without involving complicated steps such as proteinase and nitrous acid treatment for solubilizing an antigen, whereby it enables to rapidly and conveniently quantify S. mutans with a high sensitivity.

Furthermore, saliva or dental plaque is often used as a clinical sample for detecting S. mutans, but these samples have unique characteristics (for example, viscosity), and therefore, it is difficult to allow to carry out antigen-antibody reaction using these samples as they are. Accordingly, pre-treatment such as dilution of these samples in a suitable buffer and sonication of the diluted solution is generally carried out to disperse S. mutans. However, such pre-treatment leads to low concentration of the antigen and greatly lowers the sensitivity of detection of S. mutans present in a clinical sample when applied to a prior immunoassay such as reversed passive agglutination technique. The present method has the feature that it is not affected by such dilution and/or dispersion at all, because S. mutans present in the sample is filtrated and concentrated on a membrane through a process of B/F separation using the membrane. Thus, the present method enables to detect and quantify an important microorganism in dental caries, S. mutans, in a clinical sample such as saliva or dental plaque with a high sensitivity and efficiency.

Fig.1 shows graphically the relationship between concentration of S. mutans and absorbance when the present method is practiced using hydrophilic Durapore filter HVLP.

Fig.2 shows graphically the relationship between concentration of S. mutans and absorbance when the present method is practiced using Nuclepore membrane filter.

Fig.3 shows graphically the relationship between concentration of S. mutans and absorbance when the present method is practiced using Ultrafree C3GV.

Fig.4 shows graphically the relationship between concentration of S. mutans and absorbance when the present method is practiced using peroxidase-labelled MAb f89 and Multi Screen Assay System.

Fig.5 shows graphically the relationship between concentration of S. sobrinus and absorbance when the present method is practiced using peroxidase-labelled MAb g344 and Multi Screen Assay System.

Fig.6 shows graphically the relationship between absorbance and CFU on a mitis-salivarius bacitracin plate.

Fig.7 shows graphically the relationship between absorbance and CFU obtained by immune staining.

The above step (a) can be carried out as described below. A sample to be examined is suspended in a buffer suitable for allowing to quickly carry out antigen-antibody reaction so that S. mutans is dispersed homogeneously to prepare a suspension to be examined. To the suspension is added a polyclonal or monoclonal antibody (labelled or unlabelled) specifically reactive to S. mutans, and the suspension is allowed to stand at room temperature to carry out the antigen-antibody reaction. If an unlabelled specific antibody is used in this step, a labelled secondary antibody specifically reactive to the specific antibody (for example, anti-mouse IgG antibody) is then added and the suspension is allowed to stand at room temperature to carry out the antigen-antibody reaction.

The polyclonal and monoclonal antibody specifically reactive to S. mutans used in this step can be prepared by a method described in the literature. For example, the polyclonal antibody can be prepared from serum derived from a small animal such as rabbit which is immunized with S. mutans. Moreover, the monoclonal antibody can be prepared as described in Japanese Patent Publication No.177,898/1990.

The present method does not utilize agglutination, and instead utilizes filtration through a membrane filter for separating an antibody bound to S. mutans. Accordingly, it is preferred to use a monoclonal antibody having superior specificity for the reason described above. The monoclonal antibody preferred in this purpose includes, for example, MAb f89 (mouse IgG antibody) having a specific reactivity to serotypes c, e, and f of S. mutans as described in the above-mentioned Japanese Patent Publication No.177,898/1990. Of course, other monoclonal antibodies having a specific reactivity to other serotypes of the bacteria can be used to specifically detect the bacteria.

These polyclonal and monoclonal antibodies may be used alone or in combination with two or more of the antibodies.

Labelling of the antibody may be carried out using conventional labelling agents such as enzymes, fluorescent substances or radioisotopes.

The above step (b) can be carried out as follows. After the antigen-antibody reaction in the step (a) has sufficiently proceeded, the suspension is filtrated using a membrane filter capable of capturing S. mutans, and the bound antibody which has specifically reacted with S. mutans is separated from the free antibody which has not reacted with S. mutans. The membrane filter is thoroughly washed by filtration of a washing solution to wash the free antibody present on the filter off.

The membrane filter used in the separation of the bound antibody and the free antibody (B/F separation) is not limited to a particular one, and any of standard membrane filters capable of capturing a given microorganism can be employed. A preferred membrane filter is that having a less nonspecific adsorption of the free antibody and includes, for example, a porous membrane such as cellulose acetate MF-Millipore and hydrophilic Durapore (Millipore Corp.), or an isopore track etched membrane such as Nuclepore^{R} membrane filter (Nuclepore Corp.). In addition, it is preferred that the filter is treated with a commercially available blocking reagent before use. The blocking reagent is not limited to a particular one, and includes a milk protein based blocking reagent, Block Ace (Dainippon Pharmaceutical Co., Ltd.), and 1% BSA known in a literature. Also, the method of treatment may follow the method described in the literature or the protocol of the supplier.

A method for filtration includes filtration under pressure using a syringe, suction filtration under reduced pressure, centrifugation filtration or the like.

Quantification of the step (c) can be carried out according to a method well known in the art. For example, in the case of using an enzyme immunoassay, a substrate of the enzyme is added to the bound antibody captured on the filter, and the produced soluble dye or insoluble pigment is measured with a spectrophotometer or visually. On the other hand, in a radioimmunoassay, the amount of the bound antibody can be quantified by a liquid scintillation counter.

Alternatively, a sample to be examined is first filtered to capture S. mutans on a filter and a monoclonal antibody specifically reactive to S. mutans is then added to the filter. This method allows to carry out antigen-antibody reaction on the same filter.

The present invention also provides a kit for detecting and quantifying S. mutans, which kit comprises:
(i) a buffer solution containing a labelled or unlabelled antibody specific to S. mutans; and
(ii) a membrane filter for carrying out B/F separation.

In the case that the antibody specific to S. mutans is unlabelled, the kit may further contain a buffer solution containing a labelled secondary antibody specific to the former antibody. It is also desirable that the buffer solution containing such antibody further contains reagents necessary to stabilize said antibody.

Furthermore, the present kit may contain a buffer solution for dispersing and diluting a sample to be examined, and/or a washing solution for removing an antibody nonspecifically adsorbed. If an enzyme is used as a label, the kit may also contain a solution containing a substrate of said enzyme.

An antibody specific to S. mutans, label of the antibody, and membrane filter for B/F separation are as described above.

A buffer solution for use to contain an antibody includes, for example, phosphate-buffered physiological saline [PBS(-)], physiological saline, and Tris-HCl buffer containing a suitable amount of reagents for stabilizing the antibody such as bovine serum albumin (BSA), thimerosal, or sodium azide.

A buffer solution for dispersing and diluting an antibody includes PBS(-), physiological saline, Tris buffer or the like.

A washing solution for removing an antibody nonspecifically adsorbed may include distilled water, PBS(-), Tris buffer or the like, which optionally contains a suitable amount of BSA or a nonion surfactant such as Tween 20.

In the case of utilizing color development of a soluble dye by peroxidase, examples of a solution containing a substrate for the enzyme include a solution of ABTS [2,2'-azino-di-(3-ethylbenzthiazolinesulfonate(6))] dissolved in a suitable buffer as a first solution and a solution of hydrogen peroxide as a second solution. These solution are mixed in an equimolar amount when used. In the case of utilizing color development of an insoluble pigment by peroxidase, examples of the solution containing a substrate include a solution of 4-CN (4-chloro-1-naphthol) as a first solution and a solution of hydrogen peroxide as a second solution. These solutions are mixed in an equimolar amount when used. The solution containing a substrate for an enzyme may vary depending on the type of the assay system or the enzyme to be labelled, and may be prepared according to a method known in the literature.

Among the components described above, those provided in liquid are put into a suitable container to make a kit.

The present invention is further illustrated by the following Examples, but not limited thereto.

### Example 1

### Cultivation of Streptococcus mutans and oral Streptococci

S. mutans (serotype c; ATCC 25175) subcultured on Brain Heart Infusion (BHI) agar was cultivated in BHI at 37°C for 18 hours under anaerobic conditions. The cells obtained by centrifugation were washed three times with PBS(-), and suspended in PBS(-) to prepare a bacterial suspension having 0.35 of turbidity at 560nm.

The bacterial suspension thus obtained was 10-fold diluted serially with PBS(-) to prepare a series of diluted bacterial samples. The series of samples were plated on conventionally prepared mitis-salivarius agar plates (Difco Laboratories), BHI agar plates (Difco Laboratories), and Colombia CNA 5% sheep blood agar plates (Nippon Becton-Dickinson Co., Ltd.), and cultivated at 37°C for 24 hours under anaerobic conditions of 5% CO₂, 5% H₂ and 90% N₂. Subsequently, the culture was cultivated at room temperature for two days under an aerobic condition, and the number of colonies developed was counted to determine the bacterial concentration [colony forming units (cfu)/ml] of the bacterial suspension having 0.35 of turbidity at 560nm.

According to a similar method, Streptococcus salivarius (ATCC 7073) was cultivated to determine the bacterial concentration. As a result, it was found that the bacterial concentration of a bacterial suspension having 0.35 of turbidity at 560nm was 1 x 10⁷cfu/ml.

### Example 2

### Preparation of standard samples of Streptococcus mutans and oral Streptococci

A suspension of S. mutans (ATCC 25175) having 0.35 of turbidity at 560nm which was prepared in the same manner as described in Example 1 was 2-fold diluted serially with PBS(-) to prepare a series of diluted bacterial samples. These samples were used hereafter as standard samples. Standard samples of S. salivarius (ATCC 7073) were also prepared in the same manner as described above.

### Example 3

### Preparation of monoclonal antibody

Hybridoma f89 strain producing monoclonal antibody MAb f89 (mouse IgG antibody) disclosed in Japanese Patent Publication No.177,898/1990 (this strain was deposited at the Fermentation Research Institute, Agency of Industrial Science and Technology, 1-3, Higashi 1 chome, Tsukuba-shi, Ibaraki-ken, 305, Japan, on December 23, 1988 under the accession number FERM P-10464) was suspended in DMEM medium containing 10% fetal bovine serum, 0.18% NaHCO₃, 0.011% sodium pyruvate, 1 x 10⁵units/L potassium penicillin G Meiji (Meiji Seika K.K.), 0.1g titer/L streptomycin sulfate Meiji (Meiji Seika K.K.), and 2mg titer/L Fungizone^{R} (Japan Squibb K.K.) so as to obtain a cell density of 1.0 x 10⁵cells/ml. A part (10ml) of the cell suspension was charged into a 25 cm² of tissue culturing flask (Corning Co., No.25120) and incubated at 37°C in an incubator containing 5% CO₂. After four days, when proliferation of the cells reached stationary state, the cell density was about 1.6 x 10⁶ cells/ml.

Pristane (2,6,10,14-tetramethylpentadecane) (0.5ml) was administered to female BALB/c mouse (age of 6-7 weeks) intraperitoneally. Seven to ten days after the administration, the mouse was inoculated with 2 x 10⁶ cells of the above hybridoma intraperitoneally. Ten to thirty days after the inoculation, ascites fluid was collected from the mouse, and centrifuged to obtain supernatant of the ascites fluid. To the supernatant (16ml) thus obtained was added an equal volume of saturated ammonium sulfate solution, and salting-out technique was carried out. The resulting precipitation was collected by centrifugation and dissolved in PBS(-) (2.5ml). The solution containing the crude antibody thus obtained was adsorbed on a protein A column previously equilibrated with 140mM phosphate buffer (pH 8.0). Subsequently, elution was carried out serially using each of about 100ml of 140mM phosphate buffer (pH 8.0), 140mM phosphate buffer (pH 6.0), and 100mM sodium citrate buffer (pH 4.5). Fractions containing protein components eluted with 100mM sodium citrate buffer (pH 4.5) were pooled (about 24ml), and neutralized with 1M Tris-HCl buffer (pH 9.0).

The pooled and neutralized fractions were ultrafiltrated and concentrated using Centriprep^{R}-10 (W.R. Grace & Co. - Conn.) to give a concentrated antibody solution (4ml). Determination of protein concentration of this solution using a protein assay kit (Bio-Rad Co.) showed the value of 4.0mg/ml. The solution was 100-fold diluted with PBS(-), which was used as a MAb f89 antibody solution in the following Examples.

### Example 4

Hydrophilic Durapore filter HVLP (Millipore Corp.) was immersed in Block Ace (Dainippon Pharmaceutical Co., Ltd.) solution and allowed to stand at room temperature for 2 hours to carry out blocking of the filter. The filter thus treated was set onto Swinnex holder (Millipore Corp.) and washed with a washing solution [0.05% Tween 20, 10% Block Ace/PBS(-)] (2ml).

Subsequently, 40µg/ml MAb f89 solution (0.2ml) prepared in Example 3 was added to the standard sample (1ml) prepared in Example 2, and the mixture was incubated at 37°C for one hour. To the mixture was then added a solution (0.02ml) of peroxidase-labelled anti-mouse IgG (Whole Molecule) antibody (Organon Teknica Corporation) diluted 100-fold with PBS(-), and the mixture was incubated at 37°C for one hour.

After the incubation, the mixture was charged into a syringe and filtrated through the above filter set onto the holder. Immediately after the filtration, the filter was washed by filtrating the washing solution (2ml) three times in the same manner as described above.

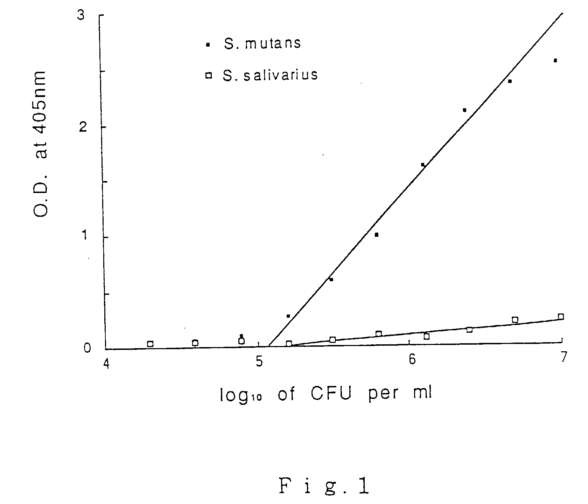

After washing, the membrane filter was removed from the holder, air-dried on a filter paper for a while, and then put into a well. To the well was added peroxidase substrate ABTS (Krikegaard & Perry Laboratories Inc.) (0.3ml), which was mixed thoroughly and allowed to react at room temperature for 30 minutes. Then, 5% SDS solution (0.1ml) was added to the well, mixed thoroughly, diluted 3.5-fold with PBS(-), and the absorbance of the resulting soluble dye was measured at 405nm. The results are shown in Fig.1. As is clear from the figure, a quantitative and proportional relationship was observed between the concentration of S. mutans and the absorbance. On the other hand, Streptococcus salivarius showed almost no absorbance.

### Example 5

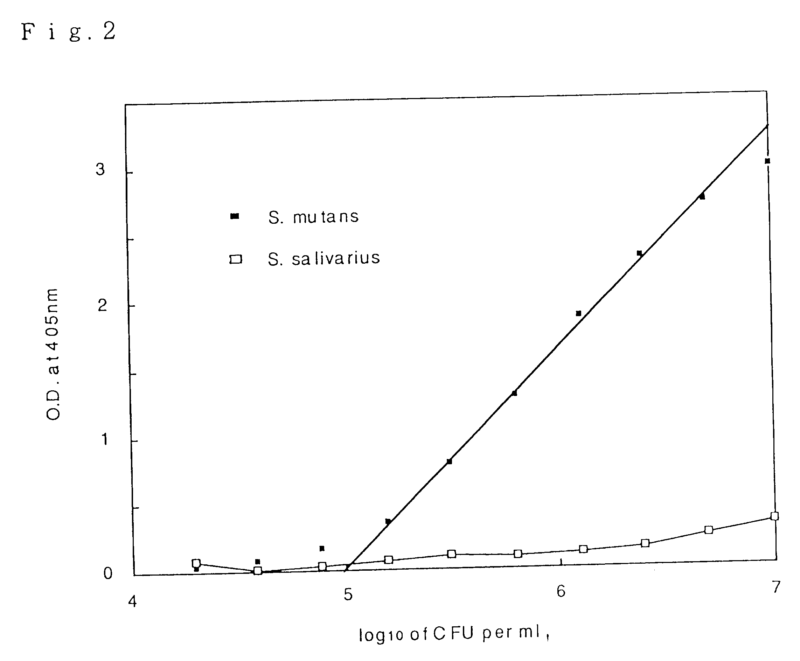

The results shown in Fig.2 were obtained in the same manner as described in Example 4, except that Nuclepore membrane filter (Nuclepore Corp.) was used rather than hydrophilic Durapore filter HVLP. Again, a quantitative and proportional relationship was observed between the concentration of S. mutans and the absorbance, and Streptococcus salivarius showed almost no absorbance.

### Example 6

MF-Millipore HAWP (Millipore Corp.) was immersed in Block Ace solution and allowed to stand at room temperature for 2 hours to carry out blocking of the filter. The filter thus treated was set onto Swinnex holder and washed with the above washing solution (2ml).

Subsequently, 40µg/ml MAb f89 solution (0.2ml) prepared in Example 3 was added to the standard sample (1ml) prepared in Example 2, and the mixture was incubated at 37°C for one hour. To the mixture was then added a solution (0.02ml) of peroxidase-labelled anti-mouse IgG antibody diluted 100-fold with PBS(-), and the mixture was incubated at 37°C for one hour.

After the incubation, the mixture was charged into a syringe and filtered through the above filter set onto the holder. After the filtration, the filter was washed by filtrating the washing solution (2ml) three times in the same manner as described above.

After washing, the outlet of the holder was capped, and 4-chloro-1-naphthol substrate (4CN Membrane Peroxidase Substrate System: Krikegaard & Perry Laboratories Inc.) (300µl) was added and allowed to react at room temperature for 30 minutes. The outlet cap of the holder was then taken off, the filter was washed two times with PBS(-) (2ml), and the resultant insoluble pigment was adsorbed onto the membrane. The membrane was then removed from the holder, air-dried on a filter paper for a while, and quantification was carried out by visually comparing the color developed.

### Example 7

Block Ace solution (400µl) was charged into the inside tube of Ultrafree C3GV (Millipore Corp.), which was allowed to stand at room temperature for 2 hours. Then, the Block Ace solution was filtrated off from the inside tube by centrifugation to accomplish blocking of the filter region. To the filter was then added a washing solution [0.05% Tween 20, 10% Block Ace/PBS(-)](400µl) and filtrated off by centrifugation.

Subsequently, 40µg/ml MAb f89 solution (0.2ml) prepared in Example 3 was added to the standard sample (1ml) prepared in Example 2, and the mixture was reacted at 37°C for one hour. To the mixture was then added a solution (0.02ml) of peroxidase-labelled anti-mouse IgG antibody diluted 100-fold with PBS(-), and the mixture was incubated at 37°C for one hour.

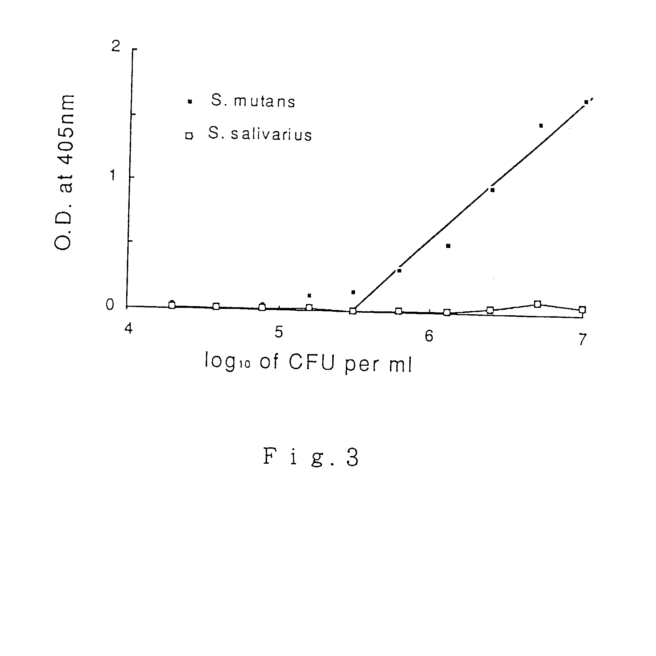

After the incubation, the mixture (400µl) was charged into the inside tube of Ultrafree C3GV which had been previously blocked as described above, and filtrated by centrifugation. Then, the above washing solution (400µl) was added to the inside tube and filtrated by centrifugation. This was repeated three times. To the inside tube was then added peroxidase substrate ABTS (0.4ml), and the mixture was reacted at room temperature for 30 minutes. Then, the mixture was filtrated by centrifugation, PBS(-) (600µl) was added to the filtrate, and the absorbance of the soluble dye produced by the reaction was measured at 405nm. The results are shown in Fig.3.

### Example 8

### Detection of Streptococcus mutans from saliva sample

Hydrophilic Durapore filter HVLP was blocked with Block Ace, set onto Swinnex holder, and washed with the above washing solution.

Saliva was collected from a subject by providing a cotton roll into the oral cavity of the subject and filtrated by centrifugation through a means prepared by packing cotton at the point of a pipet tip. Then, PBS(-) (1ml) was added to the saliva (1ml) and a 2-fold diluted sample solution was prepared. To the sample solution (1ml) was added 40µg/ml MAb f89 solution (0.2ml) prepared in Example 3, and the mixture was incubated at 37°C for one hour. To the mixture was then added a solution (0.02ml) of peroxidase-labelled anti-mouse IgG antibody diluted 100-fold with PBS(-), and the mixture was incubated at 37°C for one hour.

After the incubation, the suspension was charged into a syringe and filtrated through the above filter set onto the holder. The filter was then washed by filtrating the above washing solution (2ml) three times. After washing, the membrane filter was removed from the holder, and air-dried on a filter paper for a while. The membrane filter was put into a well. Peroxidase substrate ABTS (0.3ml) was then added to the well, mixed thoroughly, and allowed to react at room temperature for 30 minutes. Then, 5% SDS solution (0.1ml) was added to the well, mixed thoroughly, and the absorbance of the resulting soluble dye was measured at 405nm. As a result, it was found that the number of S. mutans present in saliva of the subject was 7.6 x 10⁵cfu/ml based on Fig.1.

### Example 9

### Labelling of MAb f89 with peroxidase

MAb f89 prepared in Example 3 was adjusted with 100mM phosphate buffer (pH 6.5) to 5mg/0.5ml. To the solution was added a solution prepared by dissolving S-acetylmercaptosuccinic anhydride (0.6mg) in N,N-dimethylformamide (0.01ml), and the mixture was incubated at room temperature for 30 minutes. Then, 0.1M EDTA (0.02ml), 0.1M Tris-HCl buffer (pH 7.0) (0.1ml), and 1M hydroxylamine-HCl (pH 7.0) (0.1ml) were added to the solution and the solution was incubated at 30°C for 4 minutes to terminate the reaction. Gel filtration was performed using Sephadex™ G-25 and 0.1M phosphate buffer (pH 6.0; containing 5mM EDTA) to remove unreacted components, and mercaptosuccinylated MAb f89 was recovered. The mercaptosuccinylated MAb f89 (2.3mg) was dissolved in 0.1M phosphate buffer (pH 6.0) (0.25ml) containing 5mM EDTA, to which was added a solution of maleimidated peroxidase (3.0mg) dissolved in 0.1M phosphate buffer (pH 6.0) (0.25ml), and the mixture was reacted at 4°C for 20 hours. After the reaction, purification was performed using HiLoad Superdex™ 200pg (Pharmacia) to obtain peroxidase-labelled MAb f89.

### Example 10

### Labelling of MAb g344 with peroxidase

MAb g344 was prepared in the same manner as described in Example 3 using hybridoma g344 producing monoclonal antibody MAb g344 specific to Streptococcus sobrinus (serotypes d and g) and Streptococcus mutans (serotypes c, e and f), and labelled in the same manner as described in Example 9.

### Example 11

Block Ace solution (300µl) was charged into 96-well filtration assembly MAHV N45 of Multi Screen^{R} Assay System (Millipore Corp.), and allowed to stand at room temperature for one hour. Then, filtration with suction was performed using the system and it was further washed once with the above washing solution (300µl).

Subsequently, peroxidase-labelled MAb f89 solution (40µl) prepared in Example 9 was added to the standard bacterial sample (1ml) prepared in Example 2, and the mixture was incubated at room temperature for 30 minutes.

After the incubation, the mixture (800µl) was filtrated with suction using the above 96-well filtration assembly previously blocked. Then, the above washing solution (300µl) was added and filtrated with suction. This procedure was twice repeated to perform washing.

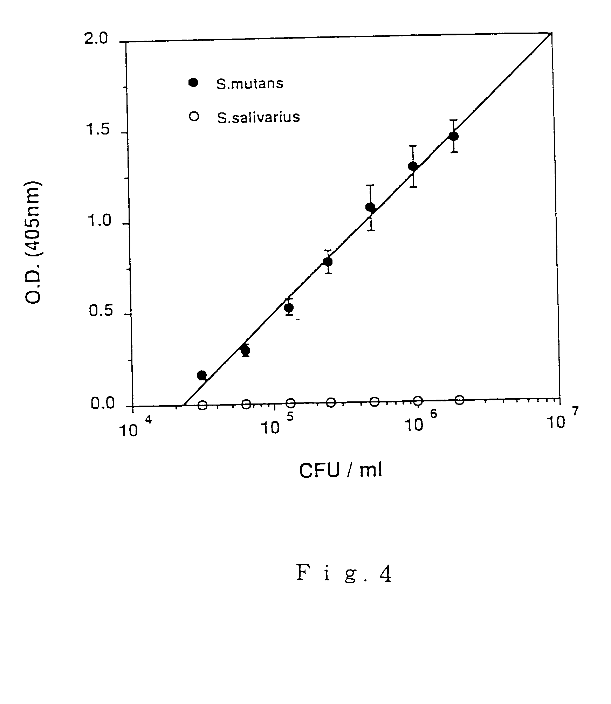

After washing, peroxidase substrate ABTS (100µl) was added to each of the wells and the mixture was reacted at room temperature for 30 minutes. Then, the mixture was filtrated with suction, the filtrate was received into 96-well EIA plate, and absorbance at 405nm was read in EIA plate reader. In addition, CFU values were determined by measuring the number of living cells in the standard bacterial sample using pour plate culture method. The relationship between EIA values and CFU values is shown in Fig.4.

### Example 12

Block Ace solution (300µl) was charged into 96-well filtration assembly MAHV N45 of Multi Screen Assay System (Millipore Corp.), and allowed to stand at room temperature for one hour. Then, filtration with suction was performed using the system and it was further washed once with the above washing solution (300µl).

Subsequently, peroxidase-labelled MAb g344 solution (40µl) prepared in Example 10 was added to the standard bacterial sample [Streptococcus sobrinus OMZ65 (serotype g; ATCC 11061); Streptococcus sangius (ATCC 10556)] (1ml) prepared in the same manner as described in Example 2, and the mixture was incubated at room temperature for 30 minutes.

After the antigen-antibody reaction, the mixture was filtrated with suction using the above 96-well filtration assembly previously blocked. Then, the above-mentioned washing solution (300µl) was added and filtrated with suction. This procedure was twice repeated to perform washing.

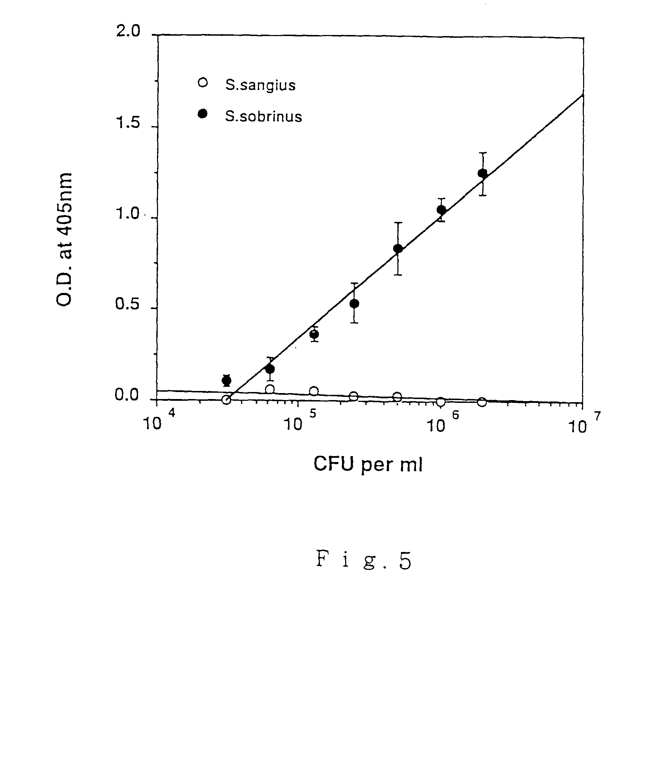

After washing, peroxidase substrate ABTS (100µl) was added to each of the wells and the mixture was reacted at room temperature for 30 minutes. Then, the mixture was filtrated with suction, the filtrate was received into a 96-well EIA plate, and absorbance at 405nm was read in an EIA plate reader. In addition, CFU values were determined by measuring the number of living cells in the standard bacterial sample using pour plate culture method. The relationship between EIA values and CFU values is shown in Fig.5.

### Example 13

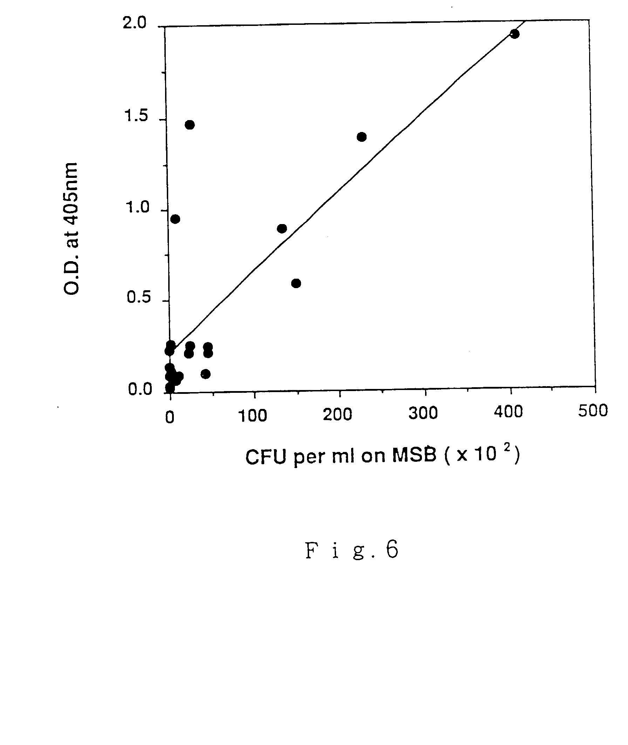

About one spoon excavator of dental plaque was collected from 21 of children at age of 2-5, suspended in PBS(-) (1ml), and sonicated to disperse uniformly. To the resulting sample solution was added peroxidase-labelled MAb f89 solution (40µl) prepared in Example 9, and the mixture was incubated at room temperature for one hour. After the incubation, the mixture (350µl) was filtrated with suction using the 96-well filtration assembly in the same manner as described in Example 11, and washed twice with the above-mentioned washing solution. Then, peroxidase substrate ABTS was added and allowed to react at room temperature for 30 minutes. After the reaction, the mixture was filtrated with suction into 96-well EIA plate, and absorbance at 405nm was read in an EIA plate reader. In addition, the sample solution was properly diluted, plated on mitis-salivarius bacitracin medium, and CFU values were measured. The relationship between EIA values and CFU values is shown in Fig.6.

### Example 14

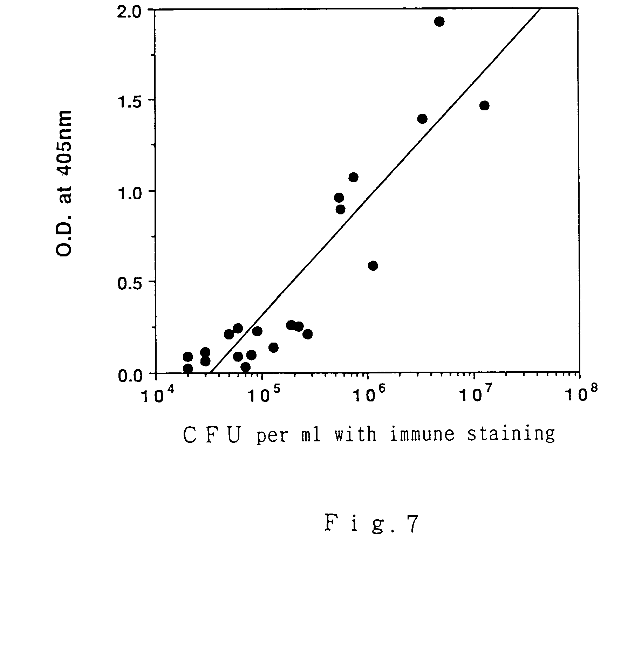

Imobilon-NC transfer membrane filter HATF (Millipore Corp.) was placed on BHI agar plate, on which the properly diluted sample solution prepared in Example 13 was plated. After incubating overnight at 37°C under an anaerobic condition, the membrane was peeled from the plate and dried at 37°C for 30 minutes. After the membrane was blocked for 30 minutes with PBST [PBS(-) supplemented with 1% BSA and 0.001% thimerosal] (200ml), peroxidase-labelled MAb f89 solution (0.4ml) was added to the PBST solution (100ml) and the mixture was incubated at room temperature for 16 hours with mildly shaking. After the incubation, the membrane was washed three times with PBST and immersed in 4-chloronaphthol substrate solution, which was allowed to stand at room temperature for 30 minutes. Then, the number of blue-stained colonies on the membrane was counted. The relationship between EIA values obtained in Example 13 and CFU values obtained by the immune staining is shown in Fig.7.

### Example 15

A kit for detecting and quantifying S. mutans was prepared and consisted of:
- A solution :: PBS(-) for dispersing and diluting a sample to be examined;
- B solution :: PBS(-) containing MAb f89 prepared in Example 3 as well as 40µg/ml thimerosal and 1mg/ml BSA;
- C solution :: POD-labelled anti-mouse IgG solution;
- Membrane :: hydrophilic Durapore filter HVLP;
- Washing solution :: PBS(-) containing 0.05% Tween 20 and 10% Block Ace;
- Coloring reagents :: the first solution : glycine buffer (pH 6.5) containing peroxidase substrate ABTS [2,2'-azino-di(3-ethylbenzthiazoline sulfate)];
the second solution: citrate buffer (pH 2.8) containing 0.02% H₂O₂.

### Example 16

A kit for detecting and quantifying S. mutans was prepared and consisted of:
- A solution :: PBS(-) for dispersing and diluting a sample to be examined;
- B solution :: PBS(-) containing peroxidase-labelled MAb f89 prepared in Example 9 as well as 40µg/ml thimerosal and 1mg/ml BSA;
- Membrane :: 96-well filtration assembly MAHV N45;
- Washing solution :: PBS(-) containing 0.05% Tween 20 and 10% Block Ace;
- Coloring reagents :: the first solution: glycine buffer (pH 6.5) containing peroxidase substrate ABTS;
the second solution: citrate buffer (pH 2.8) containing 0.02% H₂O₂.

### Effects of the Invention

As described above, the present method enables to detect S. mutans rapidly and conveniently, without the need of selective cultivation of a sample before detection, and without the problem of decrease of survival rate of bacteria caused by the time-lag between collection of a sample from a subject and its detection.

## Claims

1. A method for detecting and quantifying cariogenic bacteria which comprises the following steps:
(a) reacting Streptococcus mutans in a sample to be examined with at least one polyclonal or monoclonal antibody having a specific reactivity to the microorganism;
(b) separating the antibody bound to the microorganism from the unbound antibody by filtration through a membrane filter; and
(c) detecting the bound antibody captured on the filter.

2. The method according to Claim 1 wherein the antibody having a specific reactivity to S. mutans is a monoclonal antibody.

3. A kit for detecting and quantifying cariogenic bacteria comprising:
(i) a buffer solution containing a labelled or unlabelled antibody specific to S. mutans; and
(ii) a membrane filter for bound/free (B/F) separation.

4. The kit according to Claim 3 wherein the antibody specific to S. mutans is unlabelled and the kit further comprises a buffer solution containing a labelled secondary antibody specific to the unlabelled antibody.

5. The kit according to Claim 3 wherein the buffer solution containing the antibody further comprises a reagent necessary to stabilize the antibody.

6. The kit according to Claim 3 which further comprises a buffer solution for dispersing and diluting a sample to be examined and/or a washing solution for removing an antibody nonspecifically adsorbed.

7. The kit according to Claim 3 wherein the labelled antibody is an enzyme-labelled antibody and the kit further comprises a solution of a substrate for the enzyme.

## Patentansprüche

1. Verfahren zum Nachweis und zur Quantifizierung kariogener Bakterien, das die folgenden Schritte umfaßt:
(a) Umsetzung von in einer zu untersuchenden Probe enthaltenem Streptococcus mutans mit mindestens einem polyclonalen oder monoclonalen Antikörper mit einer spezifischen Reaktivität gegenüber dem Mikroorganismus;
(b) Trennung des an den Mikroorganismus gebundenen Antikörpers von dem ungebundenen Antikörper durch Filtration durch einen Membranfilter; und
(c) Nachweis des durch den Filter aufgefangenen Antikörpers.

2. Verfahren nach Anspruch 1, worin der Antikörper mit einer spezifischen Reaktivität gegenüber S. mutans ein monoclonaler Antikörper ist.

3. Kit für den Nachweis und die Quantifizierung kariogener Bakterien, umfassend:
(i) eine Pufferlösung enthaltend einen markierten oder unmarkierten S. mutans-spezifischen Antikörper; und
(ii) einen Membranfilter für gebunden/frei (B/F)-Trennung.

4. Kit nach Anspruch 3, worin der S. mutans-spezifische Antikörper unmarkiert ist und der Kit weiterhin eine pufferlösung umfaßt, die einen markierten zweiten Antikörper enthält, der spezifisch für den unmarkierten Antikörper ist.

5. Kit nach Anspruch 3, worin die den Antikörper enthaltende Pufferlösung weiterhin ein zur Stabilisierung des Antikörpers notwendiges Reagenz enthält.

6. Kit nach Anspruch 3, der weiterhin eine Pufferlösung zum Dispergieren und Verdünnen einer zu untersuchenden Probe und/oder eine Waschlösung zum Entfernen eines nicht-spezifisch adsorbierten Antikörpers enthält.

7. Kit nach Anspruch 3, worin der markierte Antikörper ein Enzym-markierter Antikörper ist und der Kit weiterhin eine Lösung eines Substrates für das Enzym enthält.

## Revendications

1. Procédé de détection et de détermination quantitative de bactéries cariogènes, lequel comprend les étapes suivantes :
(a) mettre à réagir Streptococcus mutans dans un échantillon à examiner avec au moins un anticorps polyclonal ou monoclonal présentant une réactivité spécifique vis-à-vis du micro-organisme ;
(b) séparer l'anticorps lié au micro-organisme de l'anticorps non lié par filtration sur un filtre à membrane ;
(c) détecter l'anticorps lié, piégé sur le filtre.

2. Procédé selon la revendication 1, dans lequel l'anticorps présentant une réactivité spécifique vis-à-vis de S. mutans est un anticorps monoclonal.

3. Kit de détection et de détermination quantitative de bactéries cariogènes, comprenant :
(i) une solution tampon contenant un anticorps marqué ou non marqué spécifique de S. mutans et
(ii)un filtre à membrane destiné à la séparation des éléments liés/libres.

4. Kit selon la revendication 3, dans lequel l'anticorps spécifique de S. mutans n'est pas marqué et le kit comprend en outre une solution tampon contenant un anticorps secondaire marqué, spécifique de l'anticorps non marqué.

5. Kit selon la revendication 3, dans lequel la solution tampon contenant l'anticorps comprend en outre un réactif nécessaire pour stabiliser l'anticorps.

6. Kit selon la revendication 3, lequel comprend en outre une solution tampon pour disperser et diluer l'échantillon à examiner et/ou une solution de lavage pour retirer un anticorps adsorbé de façon non spécifique.

7. Kit selon la revendication 3, dans lequel l'anticorps marqué est un anticorps marqué enzymatiquement et le kit comprend en outre une solution d'un substrat de l'enzyme.
